(19) Europäisches Patentamt
European Patent Office
Office européen des brevets

(11) EP 3 745 129 A1

(12) EUROPEAN PATENT APPLICATION
published in accordance with Art. 153(4) EPC

(43) Date of publication:
02.12.2020 Bulletin 2020/49

(51) Int Cl.:
G01N 33/531 (2006.01)      C07K 16/18 (2006.01)
G01N 21/64 (2006.01)       G01N 33/543 (2006.01)

(21) Application number: 19770386.1

(22) Date of filing: 22.03.2019

(86) International application number:
PCT/JP2019/012158

(87) International publication number:
WO 2019/182130 (26.09.2019 Gazette 2019/39)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.03.2018   JP 2018056175

(71) Applicant: KONICA MINOLTA, INC.
Tokyo
100-7015 (JP)

(72) Inventors:
• OTANI Makiko
  Tokyo 100-7015 (JP)
• MURAYAMA Takanori
  Tokyo 100-7015 (JP)

(74) Representative: Gille Hrabal
Brucknerstrasse 20
40593 Düsseldorf (DE)

(54) LABELED ANTIBODY DISPERSION LIQUID AND KIT FOR SPFS

(57)   An object of the present invention is to provide: a labeled antibody dispersion liquid in which a labeled antibody having a label substance bonded to an antibody is dispersed favorably when the labeled antibody is used in measurement; and a kit for SPFS. A labeled antibody dispersion liquid contains: a labeled antibody having a label substance bonded to a thiol group generated by cleaving some of disulfide bonds of an antibody molecule; and a non-ionic surfactant.

**Description**

Technical Field

The present invention relates to a labeled antibody dispersion liquid and a kit for SPFS.

Background Art

In a field such as medicine or biotechnology, in order to find a disease at an early stage, studies for detecting and quantifying a biomarker (for example, an antigen such as a specific protein) contained in human or animal blood, urine, or other biological samples (specimens) have been widely performed.

Generally, the amount of a biomarker contained in a specimen is extremely small, and its detection in clinical practice also requires accuracy. Therefore, an immunoassay for accurately detecting and quantifying a biomarker has been studied.

As the Immunoassay, a sandwich assay using an antibody for complementing an antigen (biomarker) and an antibody having a label substance for detecting the antigen bonded to the antibody (in the present invention, also referred to as a labeled antibody), a radio assay using labeling with a radioactive substance, and the like have been widely performed. In particular, the sandwich assay is excellent in detecting an extremely small amount of antigen, and is therefore a useful method for detecting and quantifying a biomarker.

Examples of a biomarker detection method using the sandwich assay include surface plasmon-field enhanced fluorescence spectroscopy (SPFS) which is a method capable of detecting an analyte with high accuracy by applying a surface plasmon resonance phenomenon utilizing bonding between a ligand and an analyte.

SPFS is a method for quantifying an analyte which is a substance to be bonded to a ligand using a sensor chip on which the ligand is immobilized. SPFS utilizes a surface plasmon light (localized field light) electric field enhancing effect that surface plasmon light (compressional wave) is generated on a surface of a metal film on a sensor chip under a condition that excitation light such as laser light emitted from a light source undergoes attenuated total reflectance (ATR) on the surface of the metal thin film, and the amount of photons possessed by the excitation light is increased by dozens of times to several hundreds of times.

In a form of the sandwich assay using SPFS, by bringing an analyte (for example, an antigen) into contact with a metal thin film on a sensor chip on which a ligand (for example, an antibody) to be specifically bonded to the analyte is immobilized, the sensor chip captures the analyte. Furthermore, an antibody to be specifically bonded to the analyte and labeled with a fluorescent substance (a labeled anti-antibody in which the label substance is a fluorescent substance. Hereinafter, also referred to as a fluorescence-labeled antibody) is brought in contact with the metal thin film. The fluorescent substance, which is a fluorescence-labeling substance bonded to the analyte captured on the metal thin film on the sensor chip by the ligand, is efficiently excited by enhanced localized field light. Therefore, by detecting a fluorescent signal derived from this fluorescent substance, it is possible to detect an extremely small amount and an extremely low concentration of the analyte.

As described above, in order to quantify an extremely small amount of antigen contained in a biological sample using SPFS, it is necessary to cause a reaction between the analyte (antigen) and the fluorescence-labeled antibody efficiently and accurately. For that purpose, the quality of the fluorescence-labeled antibody is important, and in particular, it is required for the fluorescence-labeled antibody to have high storage stability capable of retaining antibody activity even after storage for a certain period of time.

So far, studies have been made in order to prevent protein aggregation in a protein-containing formulation such as an antibody using polyoxyethylene (POE) sorbitan and polyethylene glycol (PEG) (for example, Patent Literature 1). However, in the Literature, a study on an unlabeled ordinary antibody has been made, and an aggregation-inhibiting effect of the antibody is not sufficient. In addition, there is no study on a labeled antibody in the literature.

Citation List

Patent Literature

Patent Literature 1: JP 2013-527832 A

Summary of Invention

Technical Problem

The present inventors made intensive studies on a labeled antibody having a label substance bonded to a thiol group generated by cleaving a disulfide bond contained in an antibody molecule. As a result, the present inventors have found that such a labeled antibody tends to aggregate or precipitate when being formed into a dispersion liquid because the three-dimensional structure of the antibody becomes unstable due to the cleavage of the disulfide bond which is important for maintaining the three-dimensional structure, and has lower storage stability than a dispersion liquid having an ordinary antibody dispersed therein.

In view of such circumstances, an object of the present invention is to provide a labeled antibody dispersion liquid in which a labeled antibody is dispersed favorably, and a kit for SPFS, the kit containing the dispersion liquid.

Solution to Problem

That is, the present invention provides a labeled antibody dispersion liquid and a kit for SPFS, described in, for example, [1] to [8] below.

[1] A labeled antibody dispersion liquid containing a labeled antibody having a label substance bonded to a thiol group generated by cleaving some of disulfide bonds of an antibody molecule, and a non-ionic surfactant.
[2] The labeled antibody dispersion liquid according to [1], in which the non-ionic surfactant is a polyoxyethylene-based surfactant.
[3] The labeled antibody dispersion liquid according to [1] or [2], in which the non-ionic system surfactant is a polyoxyethylene sorbitan fatty acid ester or a polyoxyethylene octyl phenyl ether.
[4] The labeled antibody dispersion liquid according to any one of [1] to [3], used for detecting an antigen contained in a sample by surface plasmon-field enhanced fluorescence spectroscopy (SPFS).
[5] The labeled antibody dispersion liquid according to any one of [1] to [4], containing 0.001 to 1% by mass of the polyoxyethylene sorbitan fatty acid ester or polyoxyethylene octyl phenyl ether with respect to 100% by mass of the labeled antibody dispersion liquid.
[6] The labeled antibody dispersion liquid according to any one of [1] to [5], in which the label substance is a fluorescent dye, a fluorescent nanoparticle, an aggregation-inducing luminescent molecule, an enzyme/coenzyme, a chemiluminescent substance, or a radioactive substance.
[7] The labeled antibody dispersion liquid according to any one of [1] to [6], in which the antibody molecule is an anti-troponin I (cTnI) antibody, an anti-troponin T (cTnT) antibody, an anti-BNP antibody, or an anti-D-dimer antibody.
[8] A kit for SPFS, the kit including: the labeled antibody dispersion liquid according to any one of [1] to [7]; and a sensor chip dedicated to surface plasmon-field enhanced fluorescence spectroscopy (SPFS).

Advantageous Effects of Invention

The present invention can provide a labeled antibody dispersion liquid having high storage stability and a kit for SPFS.

Brief Description of Drawings

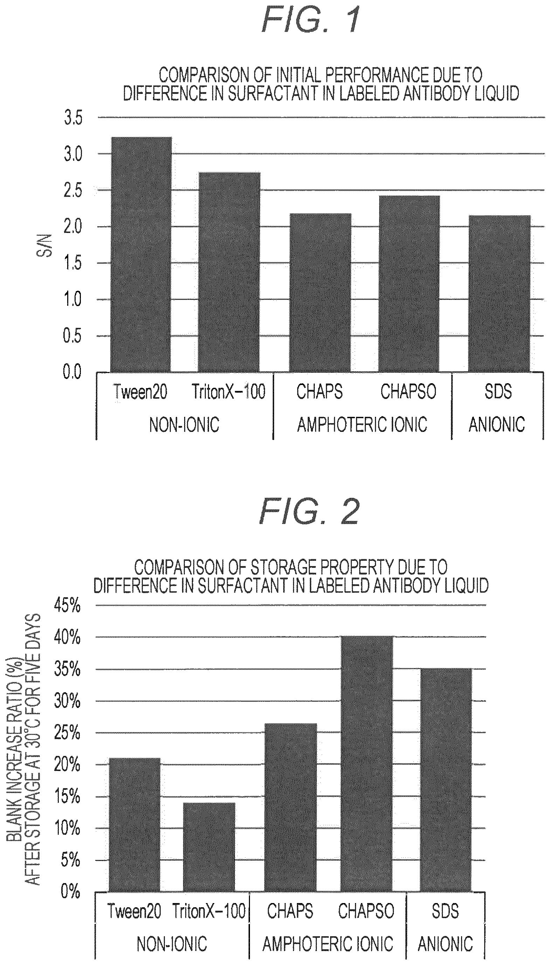

Fig. 1 is a graph illustrating results of comparing initial performance due to a difference in a surfactant in a labeled antibody dispersion liquid. The vertical axis indicates an S/N ratio, and the horizontal axis indicates the type of surfactant.
Fig. 2 is a graph illustrating results of comparing a storage property due to a difference in a surfactant in a labeled antibody dispersion liquid. The vertical axis indicates a blank increase ratio (%) after storage at 30°C for five days, and the horizontal axis indicates the type of surfactant.

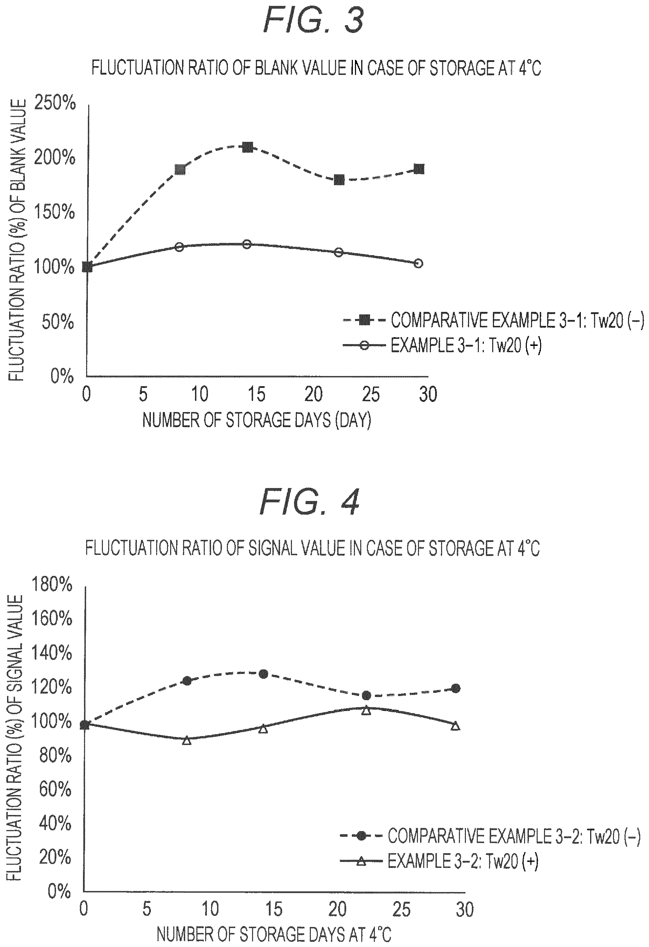

Fig. 3 is a graph illustrating a fluctuation ratio (%) of a blank value when labeled antibody dispersion liquids in Example 3-1 and Comparative Example 3-1 are stored at 4°C for 0 to 29 days. The vertical axis indicates a fluctuation ratio (%) of a blank value, and the horizontal axis indicates the number of storage days.
Fig. 4 is a graph illustrating a fluctuation ratio (%) of a signal value when labeled antibody dispersion liquids in Example 3-2 and Comparative Example 3-2 are stored at 4°C for 0 to 29 days. The vertical axis indicates a fluctuation ratio (%) of a signal value, and the horizontal axis indicates the number of storage days.

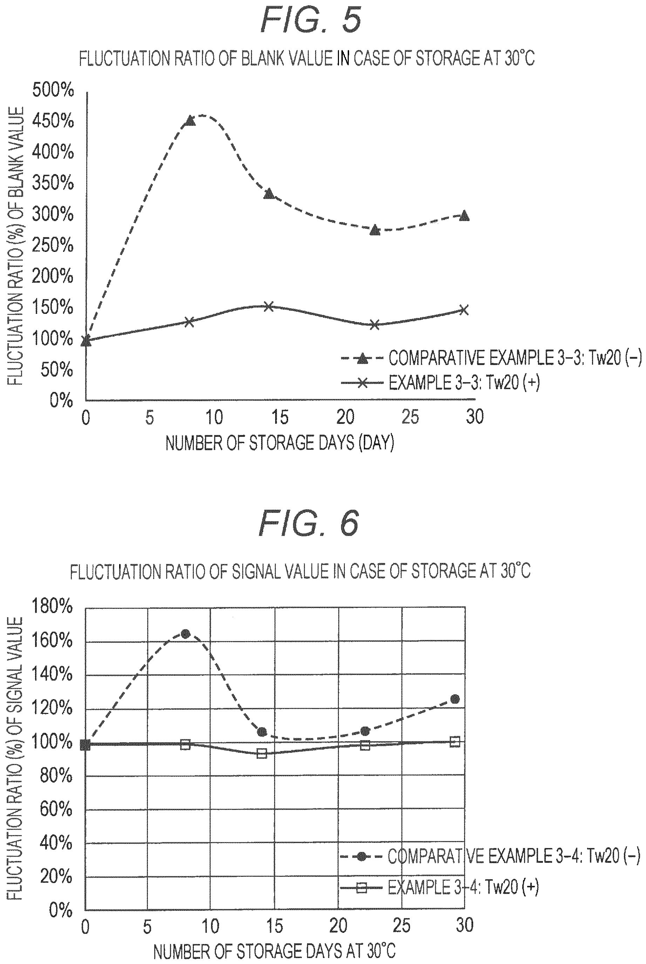

Fig. 5 is a graph illustrating a fluctuation ratio (%) of a blank value when labeled antibody dispersion liquids in Example 3-3 and Comparative Example 3-3 are stored at 30°C for 0 to 29 days. The vertical axis indicates a fluctuation ratio (%) of a blank value, and the horizontal axis indicates the number of storage days.
Fig. 6 is a graph illustrating a fluctuation ratio (%) of a signal value when labeled antibody dispersion liquids in Example 3-4 and Comparative Example 3-4 are stored at 30°C for 0 to 29 days. The vertical axis indicates a fluctuation ratio (%) of a signal value, and the horizontal axis indicates the number of storage days.

Description of Embodiments

Next, the present invention will be described specifically.

<<Labeled antibody dispersion liquid>>

A labeled antibody dispersion liquid of the present invention contains a labeled antibody having a label substance bonded to a thiol group (-SH HS-) generated by cleaving some of disulfide bonds (-S-S-) of an antibody molecule, and a non-ionic surfactant.
The labeled antibody dispersion liquid of the present invention can be used when an immunoassay such as a sandwich assay is performed.
The sandwich assay is a method for immobilizing a substance to be specifically bonded to a detection target substance in advance on a measurement area of a well plate, a sensor chip, or the like, capturing the detection target substance, and subsequently performing detection using a labeling substance having a label substance bonded to a substance to be specifically bonded to the detection target substance. Examples of a form of the sandwich assay include a sandwich immunoassay performed using a protein (antigen) as the detection target substance, using an antibody against the detection target substance as the substance to be specifically bonded to the detection target substance, and using a labeled antibody as the labeling substance.
For example, as a specific example of the sandwich immunoassay, when the antigen is cardiac troponin I (cTnI), an anti-cardiac troponin I antibody (anti-cTnI antibody) can be used as the antibody to be immobilized. As the labeled antibody, a labeled antibody having a label substance bonded to an anti-cTnI antibody can be used. In this case, the anti-cTnI antibody used as the complementing substance and the anti-cTnI antibody used as the labeled antibody are preferably antibodies that recognize different epitopes on cTnI.
The labeled antibody does not necessarily have to be a primary antibody, and may be an n-th antibody such as a secondary antibody or a tertiary antibody (n is an integer of 2 or more, preferably an integer of 2 to 4). For example, when a secondary antibody is used as the labeled antibody, the labeled antibody is preferably an anti-IgG antibody that specifically recognizes the anti-cTnI antibody.
Examples of a form of the sandwich assay include a fluorescence measurement method to be often performed for detecting an extremely small amount of a detection target substance (antigen), such as the SPFS method. Examples of a form of measurement by the SPFS method include measurement of an antigen by fluorescently labeling a detection target substance captured by a complementing substance on a surface of a measurement area of a sensor chip and detecting a fluorescent signal thereof. For such fluorescent labeling, a fluorescence-labeled antibody having a fluorescent substance bonded to an antibody may be used. Here, a dispersion liquid of such a fluorescence-labeled antibody is referred to as a fluorescence-labeled antibody dispersion liquid.
The present inventors have found that by dispersing a labeled antibody having a label substance bonded to a thiol group generated by cleaving some of disulfide bonds of an antibody molecule in a non-ionic surfactant, removal of the label substance from the labeled antibody, fragmentation of the labeled antibody, aggregation thereof, precipitation thereof, oxidation thereof, denaturation thereof, and the like are less likely to occur, and as a result, an increase in a blank value in measurement by the SPFS method is suppressed.
The labeled antibody dispersion liquid of the present invention preferably has a fluctuation ratio of a signal value within ±20% in measurement by the SPFS method before and after storage when being stored at 4°C for 29 days, or when being stored at 30°C for 14 days.
Here, the signal value is, for example, a value of a fluorescence amount obtained by measuring a specimen containing a detection target substance (antigen) when a fluorescence measurement method such as the SPFS method is performed. Meanwhile, the blank value is a value of a fluorescence amount obtained by measuring a specimen not containing a detection target substance (antigen).
A medium of the labeled antibody dispersion liquid of the present invention is preferably a buffer from a viewpoint of pH stabilization. When the medium is a buffer, examples of the buffer include an acetate buffer, a phosphate buffer, a Tris buffer, a HEPES buffer, a citrate buffer, a citrate phosphate buffer, and a borate buffer. The buffer is preferably phosphate buffered saline (PBS), Tris buffered saline (TBS), or HEPES buffered saline, which are almost isotonic with a body fluid, among the phosphate buffer, the Tris buffer, and the HEPES buffer.
The labeled antibody dispersion liquid of the present invention may contain a metal salt. The metal salt is preferably, for example, sodium chloride or potassium chloride because sodium chloride and potassium chloride are components contained in blood or the like that can be a measurement target.
The labeled antibody dispersion liquid of the present invention contains a labeled antibody at a concentration of preferably 0.5 to 10 $\mu$g/mL, more preferably 1.0 to 5.0 $\mu$g/mL.

<Labeled antibody>

The labeled antibody dispersion liquid of the present invention contains a labeled antibody having a label substance bonded to an antibody. The labeled antibody contains a labeled antibody having a label substance bonded to a thiol group generated by cleaving some of disulfide bonds of an antibody molecule.

The labeled antibody used in the present invention contains a labeled antibody having a label substance bonded to a thiol group generated by cleaving some of disulfide bonds important for maintaining the three-dimensional structure of the antibody among the disulfide bonds of the antibody. The present inventors have found that the labeled antibody as described above causes aggregation or precipitation by fragmentation or removal of a light chain or the label substance because of having an unstable three-dimensional structure. In the labeled antibody used in the present invention, a thiol group usually generated by cleavage of a disulfide bond constituting a hinge portion of the antibody is preferably bonded to the label substance. In addition, a thiol group generated by cleavage of a disulfide bond other than the hinge portion may be bonded to the label substance.

Note that when the label substance is a substance that emits fluorescence, such as a fluorescent dye described later, the label substance may be referred to as a fluorescent label substance.

(Label substance)

As the label substance used in the present invention, a substance having a functional group that can be bonded to a thiol group derived from a disulfide bond of such an antibody molecule as described above, or a substance into which a functional group is appropriately introduced by a known method only needs to be used according to a purpose of detection, and examples of the label substance include a fluorescent dyes a fluorescent nanoparticle, an aggregation-inducing luminescent molecule, an enzyme/coenzyme, a chemiluminescent substance, and a radioactive substance.

The label substance used in the present invention is preferably a fluorescent dye or a fluorescent nanoparticle from a viewpoint of being able to reduce the number of reaction steps. The fluorescent dye and the fluorescent nanoparticle preferably each contain a substance that emits fluorescence by being irradiated with predetermined excitation light or excited by utilizing an electric field effect. Here, the fluorescence has a broad sense, and includes phosphorescence having a relatively long emission lifetime in which light emission lasts and fluorescence in a narrow sense having a relatively short emission lifetime.

The type of the fluorescent dye or the like is not particularly limited.

Examples of the fluorescent dye include organic fluorescent dyes such as Alexa Fluor (registered trademark) dye series (Invitrogen Corporation), fluorescein family fluorescent dyes (Integrated DNA Technologies Inc.), polyhalofluorescein family fluorescent dyes (Applied Biosystems Japan Ltd.), hexachlorofluorescein family fluorescent dyes (Applied Biosystems Japan Ltd.), coumarin family fluorescent dyes (Invitrogen Corporation), rhodamine family fluorescent dyes (GE Healthcare Biosciences Co., Ltd.), cyanine family fluorescent dyes, indocarbocyanine family fluorescent dyes, oxazine family fluorescent dyes, thiazine family fluorescent dyes, squaraine family fluorescent dyes, chelated lanthanide family fluorescent dyes, BODIPY (registered Trademark) family fluorescent dyes (Invitrogen Corporation), naphthalene sulfonic acid family fluorescent dyes, pyrene family fluorescent dyes, and triphenylmethane family fluorescent dyes.

The fluorescent dye is not limited to the above organic fluorescent dyes. For example, a rare earth complex-based phosphor such as Eu or Tb can also be used. A rare earth complex generally has a large wavelength difference between an excitation wavelength (about 310 to 340 nm) and an emission wavelength (around 615 nm for a Eu complex and around 545 nm for a Tb complex), and usually has a relatively long fluorescence lifetime of several hundred microseconds or longer. Examples of a commercially available rare earth complex-based phosphor include ATBTA-Eu$^{3+}$.

In addition, for example, a semiconductor nanoparticle containing a II-VI group compound, a III-V group compound, or a IV group element as a component, or a core-shell type semiconductor nanoparticle containing the semiconductor nanoparticles as a core and having a shell around the core can also be used.

In addition, as the fluorescent nanoparticle, a nano-sized (diameter of 1 $\mu$m or less) particulate phosphor in which one particle can emit fluorescence with sufficient brightness can be used without any particular limitation. Typically, the fluorescent nanoparticle is a nano-sized particle having a structure in which a particle made of an organic substance or an inorganic substance is used as a base particle and a plurality of phosphors (organic fluorescent dyes or semiconductor nanoparticles) are included in the base particle and/or adsorbed on a surface of the base particle.

Examples of the fluorescent nanoparticle include an organic fluorescent dye integrated nanoparticle and an inorganic phosphor (semiconductor) integrated nanoparticle.

As the aggregation-inducing luminescent molecule, a fluorescent substance having a property of emitting strong fluorescence or increasing fluorescence intensity by increasing quantum yield by forming an aggregate by aggregation can be used without any particular limitation.

Examples of the aggregation-inducing luminescent molecule include a maleimide-based aggregation-inducing luminescent molecule, an aminobenzopyranoxanthene (ABPX)-based aggregation-inducing luminescent molecule, a benzofuro-oxazolo-carbazole-based aggregation-inducing luminescent molecule, a carborane-based aggregation-inducing luminescent molecule, a rhodamine-based aggregation-inducing luminescent molecule, a tetraphenylethylene-based aggregation-inducing luminescent molecule, a silole-based aggregation-inducing luminescent molecule, an aromatic ring-containing metal complex-based compound, a BODIPY-based boronimine complex aggregation-inducing luminescent molecule, and other hetero compounds.

Note that, for example, when blood (whole blood) is used for analysis as a specimen, in order to minimize an effect of light absorption by iron derived from blood cell components in blood, it is desirable to use a fluorescent dye, a fluorescent nanoparticle, an aggregated organic light-emitting molecule, or the like having a maximum fluorescence wavelength in a near infrared region, such as Alexa Fluor 647 (Invitrogen Corporation).

(Antibody)

The antibody used in the present invention only needs to be an antibody or an antibody fragment that specifically recognizes an antigen contained in a specimen and can be bonded to the antigen, and is appropriately selected according to an application. Examples of the antibody include a natural polyclonal antibody or monoclonal antibody, a recombinant antibody obtained by gene recombination, and fragments thereof.

For example, when troponin I (cTnI), troponin T (cTnT), CK-MB, myoglobin, H-FABP, BNP, NT-proBNP, D-dimer, or the like that can be utilized as a biomarker for myocardial infarction or the like is used as a detection target substance (antigen), an antibody that specifically recognizes these substances and is bonded to these substances can be used.

(Method for producing labeled antibody)

The labeled antibody used in the present invention is a labeled antibody having a label substance bonded to an antibody.

Specifically, for example, usually by cleaving some of disulfide bonds (-S-S-) of an antibody molecule by reduction described later for any of the above-described antibodies and bonding a label substance to two thiol groups (-SH HS-) generated from the cleaved disulfide bond, a labeled antibody can be produced.

Examples of a reducing agent that can be used for cleaving the disulfide bonds of the antibody include 2-mercaptoethanol, 3-mercapto-1,2-propanediol, glutathione (γ-L-glutamyl-L-cysteinylglycine), tris(2-carboxyethyl) phosphine hydrochloride, cysteine, and 2-mercaptoethylamine.

Bonding between the reduced antibody and the label substance can be performed by mixing the reduced antibody and the label substance in a buffer. As the buffer used at this time, a buffer that can be used as a medium for the above-described labeled antibody dispersion liquid can be used.

The labeled antibody used in the present invention can also be produced in a similar manner to a conjugate of a fluorescent substance and an antibody, the conjugate also being used in a general immunoassay. For example, by causing a reaction between a functional group introduced into a fluorescent substance and a functional group of an anti-troponin antibody in the presence of a predetermined reagent using a commercially available kit (for example, Alexa Fluor Protein Labeling Kit, Invitrogen Corporation) according to the attached protocol, a fluorescent substance-anti-troponin antibody labeled antibody can be produced.

(Non-ion surfactant)

The labeled antibody dispersion liquid of the present invention is a dispersion liquid containing a non-ionic surfactant in which the labeled antibody is dispersed.

The labeled antibody dispersion liquid of the present invention contains a non-ionic surfactant at a concentration of preferably 0.001 to 1% by mass, more preferably 0.05 to 0.5% by mass, particularly preferably 0.1 to 0.3% by mass.

The non-ionic surfactant is preferably a polyoxyethylene-based surfactant, and more preferably a polyoxyethylene sorbitan fatty acid ester or a polyoxyethylene octyl phenyl ether.

Examples of the polyoxysorbitan fatty acid ester include Tween (registered trademark) 20, Tween (registered trademark) 40, Tween (registered trademark) 60, Tween (registered trademark) 65, Tween (registered trademark) 80, and Tween (registered trademark) 85. Tween (registered trademark) 20 is particularly preferable because of its high hydrophilicity.

Examples of the polyoxyethylene octyl phenyl ether include Triton (registered trademark) X-100, Triton (registered trademark) X-114, and Triton (registered trademark) X-405. Particularly, Triton (registered trademark) X-100 is preferable because of being generally used in various conventional assays using immunological reactions.

The present inventors considered that by inclusion of a non-ionic surfactant in a labeled antibody dispersion liquid, the non-ionic surfactant would gather around a labeled antibody to make it difficult for the labeled antibody to be oxidized or denatured, and storage stability would be improved. In addition, the present inventors have found that when the non-ion surfactant is contained at a concentration within the above range, denaturation or fragmentation of the labeled antibody can be suppressed, aggregation, precipitation, and the like can be prevented more effectively, and stability during storage is further enhanced.

(Specimen)

The labeled antibody dispersion liquid of the present invention can be used when an immunoassay such as a sandwich immunoassay is performed for a specimen that may contain a detection target substance (antigen).

The specimen may be a specimen containing a detection target substance actually, or may be a specimen not containing the detection target substance actually. A target from which a specimen is collected is typically a human, but may be a non-human mammal such as a mouse, a rat, a guinea pig, a rabbit, a goat, a cat, a dog, a pig, or a monkey, which is a model animal for a human disease.

Examples of the specimen include a biologically derived substance such as blood, urine, spinal fluid, saliva, cells, tissues, organs, or preparations thereof (for example, a biopsy specimen). Blood and urine are particularly preferable as the specimen used in the present invention because blood and urine are each highly likely to contain a glycoprotein that can be utilized as a diagnostic marker.

A liquid specimen such as blood, serum, plasma, urine, spinal fluid, or saliva may be used as a specimen as it is, or may be appropriately diluted with a suitable specimen diluting liquid to be used as a specimen. A solid or semi-solid specimen such as cells, tissues, or organs can be homogenized with an appropriate buffer of about 2 to 10 times the volume of a specimen to obtain a suspension, and the suspension or a supernatant thereof can be used as it is, or can be further diluted with a specimen diluting liquid to be used as a specimen.

In a preferable example of the embodiment, blood is used as a specimen. Here, the blood may be whole blood, or serum or plasma prepared from whole blood by a known method. For example, it is preferable to use whole blood as a specimen for the purpose of performing rapid measurement, and it is preferable to remove blood cell components from whole blood by centrifugation or the like to prepare serum or plasma and then use the serum or plasma as a specimen for the purpose of accurate quantification. It is preferable to add an anticoagulant to whole blood usually at the time of blood collection. When whole blood, serum, and plasma are utilized as specimens, it is preferable to dilute the whole blood and the like to an appropriate concentration or to add a necessary reagent and the like. Therefore, such an anticoagulant, other reagents, and the like may be added to the specimen used in the present invention, if necessary.

(Antigen)

Here, a protein that is a detection target substance contained in a specimen is referred to as an antigen.

For example, troponin I (cTnI), troponin T (cTnT), CK-MB, myoglobin, H-FABP, BNP, NT-proBNP, or D-dimer that can be utilized as a biomarker for myocardial infarction or the like can be preferably selected as an antigen. When detection is performed using these substances as antigens, a specimen that can contain these antigens can be used, and a commercially available standard antigen can also be used as a control for the purpose of more accurately measuring the amount of an antigen in the specimen.

(Surface plasmon-field enhanced fluorescence spectroscopy (SPFS))

SPFS is a method for quantifying an analyte which is a substance to be bonded to a ligand using a sensor chip on which the ligand is immobilized.

In a sandwich assay using SPFS, by bringing an analyte (for example, an antigen) into contact with a metal thin film on a sensor chip on which a ligand (for example, an antibody) to be specifically bonded to the analyte is immobilized, the sensor chip captures the analyte. Furthermore, an antibody to be specifically bonded to the analyte and labeled with a fluorescent substance (a labeled antibody in which the label substance is a fluorescent substance. Hereinafter, also referred to as a fluorescence-labeled antibody) is brought in contact with the metal thin film. For the antibody used as a ligand and the antibody used as a fluorescence-labeled antibody, it is necessary to select an antibody to be specifically bonded to an analyte, but it is preferable to select antibodies that recognize different epitopes of the analyte.

In a preferable example of the embodiment of the present invention, for example, by selecting a biomarker for myocardial infarction or the like (for example, cTnI) as a detection target substance (analyte) that can be contained in a specimen, selecting an antibody to be specifically bonded to the biomarker (for example, anti-cTnI antibody) as a ligand immobilized on a sensor chip, and further performing SPFS using a fluorescence-labeled anti-cTnI antibody dispersed in the labeled antibody dispersion liquid of the present invention as a fluorescence-labeled antibody, the amount of the analyte in the specimen can be measured.

<<Kit for SPFS>>

The kit for SPFS of the present invention includes a labeled antibody dispersion liquid and a sensor chip dedicated to SPFS. The kit for SPFS of the present invention can be used when an immunoassay such as the above-described sandwich immunoassay is performed.

As a method for using the labeled antibody dispersion liquid and the sensor chip included in the kit, the sensor chip dedicated to surface plasmon-field enhanced fluorescence spectroscopy (SPFS) is set in a measuring device by the SPFS method, and an aimed detection target substance (antigen) can be detected using the labeled antibody dispersion liquid.

More specifically, for example, when cTnI is used as a detection target substance (antigen), a hydrophilic polymer layer is formed with carboxymethyl dextran (CMD) in a measurement area, and the surface plasmon-field enhanced fluorescence spectroscopy (SPFS)-dedicated sensor chip having an anti-cTnI IgG monoclonal antibody immobilized on the hydrophilic polymer layer is set in the SPFS measurement device, and cTnI can be detected using the fluorescence-labeled antibody dispersion liquid.

(Sensor chip)

The SPFS-dedicated sensor chip used in the present invention is preferably a sensor chip in which an antibody is immobilized on a surface of a metal thin film on a transparent support. In this case, the immobilized antibody is also referred to as an immobilized antibody. Examples of a method for immobilizing an antibody include a method for forming a hydrophilic polymer layer on a surface of a metal thin film, and causing a reaction of the antibody prepared so as to have an appropriate concentration at the site. The antibody immobilized on the sensor chip is appropriately selected for an antigen that is a detection target substance.

Examples

Next, the present invention will be described in more detail with reference to Examples, but the present invention is not limited thereto.

[Production Example 1]

<<Preparation of sensor chip for SPFS>>

A transparent support made of glass having a refractive index [nd] of 1.72 and a thickness of 1 mm (manufactured by OHARA Inc.: S-LAL 10) was subjected to plasma cleaning, and a chromium thin film was formed on one surface of the support by a sputtering method. Thereafter, a gold thin film, which is a metal member, was further formed on the surface by the sputtering method. The chromium thin film had a thickness of 1 to 3 nm, and the gold thin film had a thickness of 42 to 47 nm.

The support on which the gold thin film was formed was immersed in 10 mL of an ethanol solution of 10-amino-1-decanethiol prepared to 1 mM for 24 hours to form a measurement area on a surface of the gold thin film. Thereafter, the support was taken out from the ethanol solution, washed with ethanol and isopropanol, and then dried using an air gun.

<<Immobilization of antibody>>

The support on which the measurement area was formed was immersed in MES buffered saline (MES) at pH 7.4 (ionic strength: 10 mM) containing 1 mg/mL carboxymethyl dextran (CMD) having a molecular weight of 500,000, 0.5 mM N-hydroxysuccinimide (NHS), and 1 mM water-soluble carbodiimide (WSC) for one hour, and CMD was immobilized on the measurement area to form a hydrophilic polymer layer. Thereafter, the resulting product was immersed in a 1M NaOH aqueous solution for 30 minutes to hydrolyze the succinate. The CMD layer had an average film thickness of 70 nm and a density of 5.0 ng/mm$^2$.

Subsequently, the support was immersed in MES containing 50 mM NHS and 100 mM WSC for one hour, and then immersed in an anti-cTnI IgG1 monoclonal antibody (560; 2.5 μg/mL, manufactured by HyTest Ltd.) solution for 30 minutes to immobilize the antibody on the measurement area on the support. Hereinafter, the measurement area on which the antibody is immobilized is also referred to as a measurement area.

Furthermore, the solution was sent and circulated for 30 minutes with PBS containing 1% by mass bovine serum albumin (BSA) and 1 M aminoethanol to perform a non-specific adsorption prevention treatment to the measurement area.

[Experimental Example 1]

Example 1 and Comparative Example 1

<<Preparation of labeled antibody dispersion liquid>>

A fluorescence-labeled antibody was produced by the following method.

An anti-cTnI IgG monoclonal antibody (19C7; manufactured by HyTest Ltd.) was dispersed in phosphate buffered saline (PBS) at room temperature, and the concentration thereof was prepared to 1 mg/mL to obtain an antibody dispersion liquid.

A vial containing a fluorescent dye CF (registered trademark) 660R (manufactured by Biotium) was kept at room temperature, and anhydrous dimethyl sulfoxide (DMSO) was added thereto. The resulting mixture was lightly stirred to cause dispersion, and the concentration thereof was prepared to 10 mM. Then, the remaining undissolved CF (registered trademark) 660R was collected at a bottom of the vial by centrifugation for a short time, and the supernatant was collected to obtain a fluorescent dye dispersion liquid.

Subsequently, the antibody was reduced. A tris(2-carboxyethyl) phosphine (TCEP) hydrochloride solution prepared to 10 mM with PBS was added to the antibody dispersion liquid. At this time, the antibody dispersion liquid and the TCEP hydrochloride solution were mixed at a molar equivalence ratio of 1 : 10, and the resulting mixture was stirred at room temperature for 30 minutes to cause a reaction.

Thereafter, the reduced antibody dispersion liquid and the fluorescent dye dispersion liquid were mixed at a molar equivalence ratio of 1 : 10, and the resulting mixture was stirred for two hours at room temperature to cause a reaction. Thereafter, the unreacted antibody and CF (registered trademark) 660R were removed by ultrafiltration to obtain a CF (registered trademark) 660R-labeled anti-cTnI IgG monoclonal antibody (fluorescence-labeled anti-cTnI antibody) dispersion liquid. A labeling ratio was 2.7. Note that the labeling ratio was calculated from a ratio between an antibody concentration and a dye concentration after labeling, the concentrations being measured using a NanoDrop absorptiometer (manufactured by Thermo Fisher Scientific K.K.). A labeling ratio was also confirmed by the SPFS method.

The concentration was quantified by measuring the absorbance of the fluorescence-labeled anti-cTnI antibody dispersion liquid. Thereafter, the fluorescence-labeled anti-cTnI antibody dispersion liquid was diluted with a PBS solution containing surfactants of different types and concentrations, and the concentration of the fluorescence-labeled anti-cTnI antibody was prepared to 5 μg/mL. Hereinafter, the fluorescence-labeled anti-cTnI antibody dispersion liquid is referred to as a fluorescence-labeled antibody dispersion liquid.

The types and concentrations of surfactants in a fluorescence-labeled antibody dispersion liquid in each of Examples and Comparative Examples are as follows.

In each of Examples 1-1 and 1-2, a fluorescence-labeled dispersion liquid containing 0.15% by mass of Tween 20 (registered trademark) (manufactured by Nacalai Tesque, Inc.) and 0.15% by mass of Triton X-100 (registered trademark) (FUJIFILM Wako Pure Chemical Corporation), which are non-ionic surfactants, was used. In each of Comparative Examples 1-1 and 1-2, the content of each of 3-[(3-cholamidopropyl) dimethylammonio]-1-propanesulfonate (CHAPS) manufactured by Dojindo Laboratories and 3-[(3-colamidopropyl)dimethylammonio]-2-hydroxy-1-propanesulfonate (CHAPSO manufactured by Dojindo Laboratories), which are amphoteric ionic surfactants, was 0.2% by mass. In Comparative Example 1-3, a fluorescence-labeled dispersion liquid containing 0.1% by mass of sodium dodecyl sulfate (SDS) manufactured by Fujifilm Wako Pure Chemical Industries, Ltd., which is an anionic surfactant, was used.

Table 1 illustrates the types and concentrations of the surfactants used in each of Examples and Comparative Examples.

[Table 1]

Table 1 Type and concentration of surfactant

| No. | Classification | Surfactant | Concentration of surfactant in labeled antibody dispersion liquid |
|---|---|---|---|
| Example 1-1 | Non-ionic | Tween 20 | 0.15 wt% |
| Example 1-2 | | TritonX-100 | 0.15 wt% |

(continued)

| No. | Classification | Surfactant | Concentration of surfactant in labeled antibody dispersion liquid |
|---|---|---|---|
| Comparative Example 1-1 | Amphoteric ionic | CHAPS | 0.20 wt% |
| Comparative Example 1-2 | | CHAPSO | 0.20 wt% |
| Comparative Example 1-3 | Anionic | SDS | 0.10 wt% |

<<Performance of measurement>>

A PBS solution containing a commercially available cTnI control reagent (manufactured by Bio-Rad Laboratories Inc.) at a concentration of 11 ng/L was sent to a measurement area of a sensor chip for SPFS. Subsequently, the cTnI solution was removed. Thereafter, Tris buffered saline (TBS) containing 0.05% by mass of Tween 20 was sent and circulated for 10 minutes for cleaning. Thereafter, a PBS solution containing the 5 μg/mL labeled antibody dispersion liquid prepared above was sent and removed from the measurement area. Thereafter, Tris buffered saline (TBS) containing 0.05% by mass of Tween 20 was sent for cleaning.

The measurement area was filled with the PBS solution, and then the measurement area was irradiated with laser light, and a fluorescence amount was measured. This measured value was used as a signal value (S).

Meanwhile, instead of the PBS solution containing cTnI at a concentration of 11 ng/L, a PBS solution containing no cTnI (0 ng/L) was sent, and a fluorescence amount was measured by a similar procedure to the above except for the PBS solution. This measured value was used as a blank value (N).

<<Calculation of S/N ratio>>

As an index indicating initial performance of each labeled antibody dispersion liquid, an S/N ratio was calculated from the signal value (S) and the blank value (N) obtained as described above using the following Formula (I).

$$(\text{Signal value at cTnI concentration of 11 ng/L})/(\text{Blank value at cTnI concentration of 0 ng/L}) \ldots \text{Formula (I)}$$

<<Calculation of blank increase ratio (%)>>

As a study of storage property when each labeled antibody dispersion liquid was stored at 30°C for five days, a blank increase ratio (%) was calculated using the following Formula (II).

$$\{(\text{Blank value when labeled antibody dispersion liquid is stored at 30°C for five days})/(\text{Blank value immediately after labeled antibody dispersion liquid is produced}) - 1\} \times 100 \ldots \text{Formula (II)}$$

Table 2 illustrates classification of the surfactants in the labeled antibody dispersion liquid used in each of Examples and Comparative Examples, an initial performance value (S/N ratio), and results of a storage property of each dispersion liquid when the labeled antibody dispersion liquid is stored at 30°C for five days.

[Table 2]

Table 2 Difference in type of surfactant in labeled antibody dispersion liquid and storage property

| No. | Classification | Surfactant | Initial performance | | | Storage property | | |
|---|---|---|---|---|---|---|---|---|
| | | | Blank | Signal (cTnI: 11 ng/L) | S/N | Blank_ 0 day | Blank 30°C 5 day | Blank increase ratio |
| Example 1-1 | Non-ionic | Tween 20 | 7960 | 23855 | 3.24 | 7360 | 8906 | 21% |
| Example 1-2 | | TritonX-100 | 10139 | 27768 | 2.74 | 10139 | 11558 | 14% |
| Comparative Example 1-1 | Amphoteric ionic | CHAPS | 11060 | 24011 | 2.17 | 11060 | 13979 | 26% |
| Comparative Example 1-2 | | CHAPSO | 10520 | 25493 | 2.42 | 10520 | 14728 | 40% |
| Comparative Example 1-3 | Anionic | SDS | 12666 | 27159 | 2.14 | 12666 | 17099 | 35% |

Fig. 1 is a graph illustrating results of comparing initial performance due to a difference in a surfactant in a labeled antibody dispersion liquid. The vertical axis indicates an S/N ratio, and the horizontal axis indicates the type of surfactant.

Fig. 2 is a graph illustrating results of comparing a storage property due to a difference in a surfactant in a labeled antibody dispersion liquid. The vertical axis indicates a blank increase ratio after storage at 30°C for five days, and the horizontal axis indicates the type of surfactant.

[Experimental example 2]

Example 2 and Comparative Example 2

<<Preparation of labeled antibody dispersion liquid>>

A labeled antibody dispersion liquid was prepared in a similar manner to Experimental Example 1 except that non-ionic surfactants Tween 20 and Triton X-100 were used in the amounts illustrated in Table 3.

<<Performance of measurement/Calculation of S/N ratio and blank increase ratio (%)>>

Measurement was performed in a similar manner to Experimental Example 1, and an S/N ratio and a blank increase ratio (%) were calculated from the obtained signal value and blank value.

Table 3 illustrates the type and concentration of a non-ion surfactant, an initial performance value (S/N ratio), and results of a storage property of each dispersion liquid when the dispersion liquid was stored at 30°C for five days.

[Table 3]

Table 3 Difference in type of non-ionic surfactant in labeled antibody dispersion liquid and storage property

| No. | Surfactant | Concentration of surfactant in labeled antibody dispersion liquid | Initial performance | | | Storage property | | |
|---|---|---|---|---|---|---|---|---|
| | | | Blank | Signal (cTnI: 11 ng/L) | S/N | Blank_ 0 day | Blank_ 30°C 5 days | Blank increase ratio |
| Comparative Example 2-1 | Tween 20 | 0 | 11305 | 27507 | 2.4 | 11305 | 14985 | 33% |
| Example 2-1 | | 0.10 wt% | 7549 | 23200 | 3.1 | 7549 | 9306 | 23% |
| Example 2-2 | | 0.15 wt% | 7360 | 23855 | 3.2 | 7360 | 8906 | 21% |
| Example 2-3 | | 0.30 wt% | 7242 | 20885 | 2.9 | 7242 | 8183 | 13% |
| Example 2-4 | | 0.50 wt% | 7382 | 19851 | 2.7 | 7382 | 8563 | 16% |
| Comparative Example 2-2 | TritonX-100 | 0 | 11305 | 27507 | 2.4 | 11305 | 14985 | 33% |
| Example 2-5 | | 0.10 wt% | 10947 | 28283 | 2.6 | 10947 | 13536 | 24% |
| Example 2-6 | | 0.15 wt% | 10139 | 27768 | 2.7 | 10139 | 11356 | 12% |
| Example 2-7 | | 0.30 wt% | 9716 | 26242 | 2.7 | 9716 | 10979 | 13% |
| Example 2-8 | | 0.50 wt% | 9132 | 22392 | 2.5 | 9132 | 10319 | 13% |

[Experimental Example 3]

Example 3 and Comparative Example 3

<<Preparation of labeled antibody dispersion liquid>>

A fluorescence-labeled antibody dispersion liquid was prepared in a similar manner to Experimental Example 1 except that the reduced antibody dispersion liquid and the fluorescent dye dispersion liquid were mixed at a molar equivalence ratio of 1 : 20. Note that for adjusting the concentration of the fluorescence-labeled antibody dispersion liquid, 0.15% Tween 20-PBS was used in Examples, and PBS was used in Comparative Examples. A labeling ratio was 5.1.

<<Performance of measurement/Calculation of S/N ratio and blank increase ratio (%)>>

Similar operation to Experimental Example 1 was performed except that a PBS solution containing a commercially available cTnI control reagent (manufactured by Bio-Rad Laboratories Inc.) at a concentration of 9.5 ng/L was sent to a measurement area.
Note that it is confirmed that Experimental Examples 1 and 2 and Experimental Example 3 have no difference in storage property although having different labeling ratios. The inventor considers that a reason for this is that reduction (labeling) damages the antibody to the same extent because the antibody reduction conditions are the same.
Table 4 illustrates the concentration of Tween 20 and measurement results of a blank value (0 ng/L) and a signal value (9.5 ng/L) at each number of storage days in a case of storage at 4°C or 30°C for 0 to 29 days.

[Table 4]

Table 4 Difference in concentration of Tween 20 in labeled antibody dispersion liquid and measurement result of blank value/signal value at each storage temperature and each number of storage days

| | Concentration (% by mass) of Tween 20 in labeled antibody dispersion liquid | Storage temperature (°C) | Specimen concentration cTI: 0 ng/L cTI: 9.5 ng/L | Blank value/signal value at each number of storage days | | | | |
|---|---|---|---|---|---|---|---|---|
| | | | | 0 day | 8 days | 14 days | 22 days | 29 days |
| Comparative Example 3-1 | (-) 0% | 4°C | 0 ng/L | 49 | 93 | 103 | 88 | 93 |
| Comparative Example 3-2 | (-) 0% | 4°C | 9.5 ng/L | 313 | 393 | 405 | 367 | 380 |
| Comparative Example 3-3 | (-) 0% | 30°C | 0 ng/L | 49 | 223 | 165 | 136 | 147 |
| Comparative Example 3-4 | (-) 0% | 30°C | 9.5 ng/L | 313 | 520 | 334 | 333 | 392 |
| Example 3-1 | (+) 0.15% | 4°C | 0 ng/L | 99 | 117 | 120 | 114 | 103 |
| Example 3-2 | (+) 0.15% | 4°C | 9.5 ng/L | 406 | 368 | 399 | 442 | 406 |
| Example 3-3 | (+) 0.15% | 30°C | 0 ng/L | 99 | 130 | 153 | 124 | 145 |
| Example 3-4 | (+) 0.15% | 30°C | 9.5 ng/L | 406 | 403 | 381 | 400 | 408 |

Using the blank value and the signal value, an S/N ratio was calculated in a similar manner to Experimental Example 1. Table 5 illustrates the concentration of Tween 20 and an S/N ratio at each number of storage days in a case of storage at 4°C or 30°C for 0 to 29 days.

[Table 5]

Table 5 Difference in concentration of Tween 20 in labeled antibody dispersion liquid and S/N result at each storage temperature and each number of storage days

| | Concentration (% by mass) of Tween 20 in labeled antibody dispersion liquid | Storage temperature (°C) | Specimen concentration cTI: 0 ng/L cTI: 9.5 ng/L | S/N | | | | |
|---|---|---|---|---|---|---|---|---|
| | | | | 0 day | 8 days | 14 days | 22 days | 29 days |
| Comparative Example 3-1 | (-) 0% | 4°C | 0 ng/L | 6.4 | 4.2 | 3.9 | 4.2 | 4.1 |
| Comparative Example 3-2 | (-) 0% | 4°C | 9.5 ng/L | | | | | |
| Comparative Example 3-3 | (-) 0% | 30°C | 0 ng/L | 6.4 | 2.3 | 2.0 | 2.5 | 2.7 |
| Comparative Example 3-4 | (-) 0% | 30°C | 9.5 ng/L | | | | | |
| Example 3-1 | (+) 0.15% | 4°C | 0 ng/L | 4.1 | 3.1 | 3.3 | 3.9 | 3.9 |
| Example 3-2 | (+) 0.15% | 4°C | 9.5 ng/L | | | | | |
| Example 3-3 | (+) 0.15% | 30°C | 0 ng/L | 4.1 | 3.1 | 2.5 | 3.2 | 2.8 |
| Example 3-4 | (+) 0.15% | 30°C | 9.5 ng/L | | | | | |

<<Calculation of fluctuation ratios (%) of blank value and signal value>>

Based on a blank value of the labeled antibody dispersion liquid after storage for 0 day, a blank value (%) at each number of storage days in a case of storage at 4°C or 30°C for 0 to 29 days was calculated by the following Formula (III).

$$(\text{Blank value of labeled antibody dispersion liquid after storage of 8 to 29 days})/(\text{Blank value of labeled antibody dispersion liquid after storage of 0 day}) \times 100 \ldots \text{Formula (III)}$$

A fluctuation ratio (%) of a signal value was calculated in a similar manner by changing the blank value of the Formula (III) to a signal value.

Table 6 illustrates the concentration of Tween 20 and fluctuation ratios (%) of a blank value and a signal value at each number of storage days in a case of storage at 4°C or 30°C for 0 to 29 days.

[Table 6]

Table 6 Difference in concentration of Tween 20 in labeled antibody dispersion liquid and fluctuation ratios (%) of blank value and signal value at each storage temperature based on values after storage for 0 day

| | Concentration (% by mass) of Tween 20 in labeled antibody dispersion liquid | Storage temperature (°C) | Specimen concentration cTI: 0 ng/L cTI: 9.5 ng/L | Fluctuation ratios (%) of blank value and signal value based on values after storage for 0 day | | | | |
| | | | | 0 day | 8 days | 14 days | 22 days | 29 days |
|---|---|---|---|---|---|---|---|---|
| Comparative Example 3-1 | (-) 0% | 4°C | 0 ng/L | 100% | 189% | 210% | 180% | 190% |
| Comparative Example 3-2 | (-) 0% | 4°C | 9.5 ng/L | 100% | 126% | 129% | 117% | 122% |
| Comparative Example 3-3 | (-) 0% | 30°C | 0 ng/L | 100% | 456% | 336% | 277% | 300% |
| Comparative Example 3-4 | (-) 0% | 30°C | 9.5 ng/L | 100% | 166% | 107% | 106% | 125% |
| Example 3-1 | (+) 0.15% | 4°C | 0 ng/L | 100% | 118% | 121% | 115% | 104% |
| Example 3-2 | (+) 0.15% | 4°C | 9.5 ng/L | 100% | 91% | 98% | 109% | 100% |
| Example 3-3 | (+) 0.15% | 30°C | 0 ng/L | 100% | 131% | 154% | 125% | 146% |
| Example 3-4 | (+) 0.15% | 30°C | 9.5 ng/L | 100% | 99% | 94% | 99% | 101% |

Figs. 3 to 6 illustrate graphs of fluctuation ratios (%) of a blank value and a signal value based on a blank value and a signal value after storage for 0 day, the fluctuation ratios (%) being illustrated in Table 6 above.

**Claims**

1. A labeled antibody dispersion liquid comprising: a labeled antibody having a label substance bonded to a thiol group generated by cleaving some of disulfide bonds of an antibody molecule; and a non-ionic surfactant.

2. The labeled antibody dispersion liquid according to claim 1, wherein the non-ionic surfactant is a polyoxyethylene-based surfactant.

3. The labeled antibody dispersion liquid according to claim 1 or 2, wherein the non-ionic system surfactant is a polyoxyethylene sorbitan fatty acid ester or a polyoxyethylene octyl phenyl ether.

4. The labeled antibody dispersion liquid according to any one of claims 1 to 3, used for detecting an antigen contained in a sample by surface plasmon-field enhanced fluorescence spectroscopy (SPFS).

5. The labeled antibody dispersion liquid according to any one of claims 1 to 4, comprising 0.001 to 1% by mass of the polyoxyethylene sorbitan fatty acid ester or polyoxyethylene octyl phenyl ether with respect to 100% by mass of the labeled antibody dispersion liquid.

6. The labeled antibody dispersion liquid according to any one of claims 1 to 5, wherein the label substance is a fluorescent dye, a fluorescent nanoparticle, an aggregation-inducing luminescent molecule, an enzyme/coenzyme, a chemiluminescent substance, or a radioactive substance.

7. The labeled antibody dispersion liquid according to any one of claims 1 to 6, wherein the antibody molecule is an anti-troponin I (cTnI) antibody, an anti-troponin T (cTnT) antibody, an anti-BNP antibody, or an anti-D-dimer antibody.

8. A kit for SPFS, the kit comprising: the labeled antibody dispersion liquid according to any one of claims 1 to 7; and a sensor chip dedicated to surface plasmon-field enhanced fluorescence spectroscopy (SPFS).

## FIG. 1

COMPARISON OF INITIAL PERFORMANCE DUE TO
DIFFERENCE IN SURFACTANT IN LABELED ANTIBODY LIQUID

## FIG. 2

COMPARISON OF STORAGE PROPERTY DUE TO
DIFFERENCE IN SURFACTANT IN LABELED ANTIBODY LIQUID

# FIG. 3

FLUCTUATION RATIO OF BLANK VALUE IN CASE OF STORAGE AT 4°C

# FIG. 4

FLUCTUATION RATIO OF SIGNAL VALUE IN CASE OF STORAGE AT 4°C

## FIG. 5

FLUCTUATION RATIO OF BLANK VALUE IN CASE OF STORAGE AT 30°C

- --▲-- COMPARATIVE EXAMPLE 3-3: Tw20 (−)
- --×-- EXAMPLE 3-3: Tw20 (+)

## FIG. 6

FLUCTUATION RATIO OF SIGNAL VALUE IN CASE OF STORAGE AT 30°C

- --●-- COMPARATIVE EXAMPLE 3-4: Tw20 (−)
- --□-- EXAMPLE 3-4: Tw20 (+)

## INTERNATIONAL SEARCH REPORT

| International application No. |
| --- |
| PCT/JP2019/012158 |

### A. CLASSIFICATION OF SUBJECT MATTER
Int.Cl.　　G01N33/531(2006.01)i, C07K16/18(2006.01)i, G01N21/64(2006.01)i,
　　　　　G01N33/543(2006.01)i

According to International Patent Classification (IPC) or to both national classification and IPC

### B. FIELDS SEARCHED

Minimum documentation searched (classification system followed by classification symbols)
Int.Cl. G01N33/531, C07K16/18, G01N21/64, G01N33/543

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2000-329765 A (MATSUSHITA ELECTRIC INDUSTRIAL CO., LTD.) 30 November 2000, claim 1, paragraphs [0011], [0014], [0015], fig. 1 (Family: none) | 1–8 |
| A | JP 4-48266 A (TDK CORPORATION) 18 February 1992, claim 1 (Family: none) | 1–8 |
| A | JP 2017-172975 A (TOSOH CORP.) 28 September 2017, claim 1, paragraphs [0002], [0017] (Family: none) | 1–8 |
| A | CN 106872686 A (GUANGDONG SHUNDE INDUSTRIAL DESIGN INSTITUTE) 20 June 2017, claim 1, paragraph [0001], example 1 (Family: none) | 1–8 |
| A | WO 2018/051863 A1 (KONICA MINOLTA, INC.) 22 March 2018, paragraph [0017] (Family: none) | 1–8 |

☐ Further documents are listed in the continuation of Box C. ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 14 June 2019 (14.06.2019) | 25 June 2019 (25.06.2019) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013527832 A